# EUROPEAN PATENT APPLICATION

(11) **EP 2 002 858 A2**
(43) Date of publication of application: **17.12.2008**
(21) Application number: 08251999.2
(22) Date of filing: 10.06.2008
(51) Int. Cl.: A61M 25/09, A61M 25/00

(54) **Single use deflectable stylet**

(30) Priority: 15.06.2007 US 934844 P
(71) Applicant: CathRx Ltd, Eveleigh, New South Wales 1430 (AU)
(72) Inventor: Partlett, Matthew, Allawah, New South Wales 2218 (AU); Ogle, David, Cowan, New South Wales 2081 (AU)
(74) Representative: Nettleton, John Victor

(57) **Abstract**

A single use, deflectable stylet 10 for use in a catheter includes a deflectable portion 12 of a flexible material. An actuator 14 is attached to the deflectable portion 12, manipulation of the actuator 14 relative to the deflectable portion 12 causing deflection of the deflectable portion 12. The stylet 10 further includes a stiffening element 16 with the deflectable portion 12 being associated with a distal region of the stiffening element 16, the stiffening element 16 being sufficiently stiff in torsion to convert proximal rotation into substantially equivalent distal rotation.

## Description

### Cross-Reference to Related Applications

The present application claims priority from United States of America Provisional Patent Application No 60/934,844 filed on 15 June 2007, the contents of which are incorporated herein by reference.

### Field

This invention relates, generally, to catheters and, more particularly, to a single use deflectable stylet for use in a catheter, to a method of fabricating a stylet and to a catheter including the stylet.

### Background

In heart procedures, a catheter, be it a diagnostic catheter or a therapeutic catheter, is inserted via an introducer into the vasculature of a patient. Normally, the catheter is inserted through the femoral vein of a patient and is steered to a site in a patient's heart at which a diagnostic and/or therapeutic procedure is to be carried out. At the site, it is necessary to deflect a tip of the catheter to bring electrodes of the catheter into contact with heart wall tissue to effect diagnosis or therapy.

Thus, a tip of the catheter needs to be deflectable. This is achieved, in the case of the Applicant's catheter, by inserting a stylet into a lumen of an electrode sheath of the catheter. The electrode sheath is manufactured according to the Applicant's manufacturing technique and has an unimpeded lumen. This is due to the fact that electrical conductors for electrodes of the electrode sheath are generally embedded in a wall of the electrode sheath.

The catheter is also manufactured with a low-cost handle. The low-cost handle is achieved by not having electrical conductors or connectors arranged in the catheter. Rather, the electrical conductors pass through the body of the catheter and are connected directly to equipment for communicating with the electrodes of the catheter. It would therefore be beneficial to have a stylet which is also a low-cost item to enable it to be used once only without significantly increasing expenses of an institution using the catheters.

### Summary

According to a first aspect of the invention, there is provided a single use, deflectable stylet for use in a catheter, the stylet including
a deflectable portion of a flexible material;
an actuator attached to the deflectable portion, manipulation of the actuator relative to the deflectable portion causing deflection of the deflectable portion; and
a stiffening element with the deflectable portion being associated with a distal region of the stiffening element, the stiffening element being sufficiently stiff in torsion to convert proximal rotation into substantially equivalent distal rotation.

Preferably, the deflectable portion is of a synthetic plastics material. Further, the stiffening element may be of metal. The metal may be a low cost steel material such as a stainless steel material or it may be aluminium, tungsten , or the like.

The deflectable portion may be an elongate tubular body member defining a passage. The tubular body member may define an axially extending cutaway region in a wall of the body member to facilitate deflection of a distal part of the body member.

The cutaway region may subtend an angle of greater than 90° but less than 270°. Typically the cutaway region may subtend an angle of approximately 170° to 190°.

In this specification, the term "subtend", unless the context clearly indicates otherwise, is used in the sense of forming or marking the limits of the angle.

In an embodiment, the cutaway region may be closed off by a cage defined by a plurality of longitudinally spaced, transversely extending slots. stead of, or in addition to, the cutaway region, a plurality of longitudinally spaced, transversely extending slots may be defined in the distal part of the tubular body member to facilitate deflection of the distal part of the body member.

In one embodiment, the stiffening element may be a tube received in the passage of the tubular body member. In another embodiment, the stiffening element may be a sleeve received over the tubular body member.

In a further embodiment, the stiffening element may be a tube and the deflectable portion may constitute a tip portion mounted on a distal end of the tube. In this embodiment too, the deflectable portion may comprise an elongate tubular body member defining an axially extending cutaway region in a wall of the body member to facilitate deflection of the distal part of the body member.

The actuator may be a pull wire extending through the tube and the tubular body member, a distal end of the pull wire being fast with a distal end of the tubular body member.

In still a further embodiment, the deflectable portion may comprise an elongate, planar element having a mounting formation at its proximal end for mounting to the distal end of the tube. In this embodiment, the actuator may be a pull wire extending through the tube and over the planar element with a distal end of the pull wire being fast with a distal end of the planar element.

According to a second aspect of the invention, there is provided a single use, deflectable stylet for use in a catheter, the stylet including
an elongate, tubular body member of a synthetic plastics material, the plastics material being of a type which permits deflection of at least a part of the body member but which is sufficiently stiff in torsion to convert rotation at a proximal end of the body member into substantially equivalent rotation at a distal end of the body member, the body member defining a lumen;
an axially extending cutaway region defined in a wall of the body member at a distal part of the body member to facilitate deflection of the distal part of the body member, the cutaway region subtending an angle of greater than 90° but less than 270°; and
an actuator received in the lumen of the body member, a distal end of the actuator being fast with a distal end of the body member and relative displacement between the body member and the actuator causing deflection of the body member about the cutaway region of the body member.

The body member may be of a thermoplastic material. The thermoplastic material may be a polyetheretherketone (PEEK) material or a polyimide material.

The cutaway region may be formed with the actuator in position in the lumen of the body member. The cutaway region may be an inwardly crenated region formed in the wall of the body member. The cutaway region may be closed off by a cage comprising a plurality of longitudinally spaced, transversely extending slots.

The stylet may include a mounting assembly for mounting to a handle body of a catheter. The mounting assembly may include an anchoring element mountable to the handle body and a carrier displaceably arranged relative to the anchoring element, the actuator being mounted fast with one of the anchoring element and the carrier with another component being mounted fast with the other of the carrier and the anchoring element so that relative displacement of the actuator and the other component causes deflection of the deflectable portion.

Depending on the embodiment, the "other component" may be the tubular body member itself or, where the tubular body member is mounted on a distal end of the stiffening member, the stiffening member.

According to a third aspect of the invention, there is provided a method of fabricating a deflectable stylet the method including
providing an elongate tubular body member defining a lumen;
inserting an actuator into the lumen of the body member; and
using the actuator as a guide formation, forming an inwardly crenated, axially extending cutaway portion in a wall of the tubular member to form a bend-enhancing region about which the body member is able to deflect.

The method may include forming the cutaway portion with a subtended angle greater than 90° but less than 270°.

Further, the method may include securing a distal end of the actuator fast with a distal end of the body member.

The method may include providing a stiffening member for the stylet. The stiffening member may be a tube and the method may include mounting the tubular body member on a distal end of the tubular member. Instead, the stiffening member may be a tube and the method may include inserting the tube into the lumen of the body member. Still further, the stiffening member may be a tube and the method may include inserting the body member into a passage of the tube.

According to a fourth aspect of the invention, there is provided a catheter which includes
a catheter handle having a handle body;
an electrode sheath extending from a distal end of the handle, the electrode sheath defining a lumen; and
a single use, deflectable stylet, as described above, received in the lumen of the electrode sheath.

The catheter may include a deflection control mechanism which is displaceably carried on the handle body, the carrier of the mounting assembly being secured to the displacement control mechanism and the anchoring formation being secured to a proximal end of the handle body.

A projection control mechanism may be carried on the handle body, the electrode sheath being carried on the projection control mechanism and the projection control mechanism being operable to extend and retract the electrode sheath relative to the stylet.

### Brief Description of Drawings

Fig. 1 shows a three dimensional view of an embodiment of a single use, deflectable stylet;
Fig. 2 shows a three dimensional view of a distal part of the stylet of Fig. 1;
Fig. 3 shows a three dimensional view of another embodiment of a single use, deflectable stylet;
Fig. 4 shows a three dimensional view of a distal part of the stylet of Fig. 3;
Fig. 5 shows a three dimensional view of a deflectable part of the stylet of Fig. 3;
Fig. 6 shows a side view of a part of a further embodiment of a single use, deflectable stylet;
Fig. 7 shows a sectional end view taken along line VII-VII in Fig. 6;
Fig. 8 shows a three dimensional view of an embodiment of a catheter including a single use, deflectable stylet;
Fig. 9 shows a sectional side view of a part of the catheter; and
Fig. 10 shows a side view of a part of a still a further embodiment of a single use, deflectable stylet.

### Detailed Description of Exemplary Embodiments

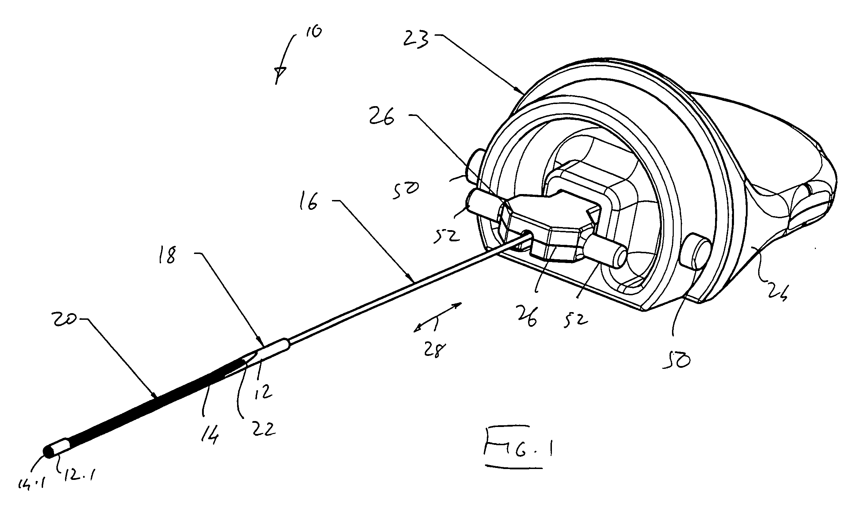

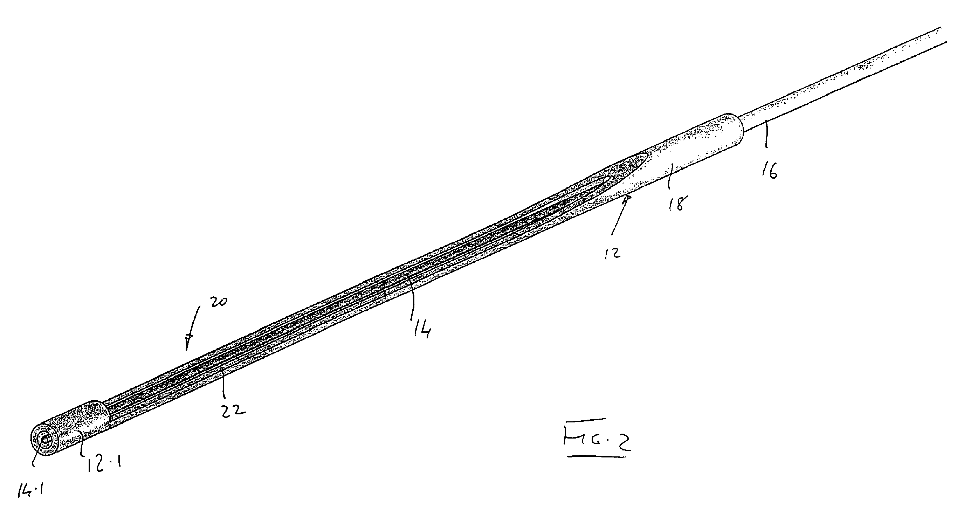

In Figs. 1 and 2, reference numeral 10 generally designates an embodiment of a single use, deflectable stylet 10. The stylet 10 includes a deflectable portion 12 of a flexible material. An actuator, in the form of a pull wire, 14 is received through the deflectable portion 12. A distal end 14.1 of the pull wire 14 is fast with a distal end 12.1 of the deflectable portion 12.

The stylet 10 includes a stiffening element in the form of a tube 16. In this embodiment, the deflectable portion 12, which comprises an elongate tubular member 18, is mounted on a distal end of the tube 16. The tube 16 is of metal such as stainless steel.

The tubular body member 18 of the deflectable portion 12 is of a thermoplastics material. For example, the tubular body member 12 can be made of a polyetheretherketone (PEEK) plastics or a polyimide plastics material.

The tubular body member 18 defines a bend-enhancing region 20. The bend-enhancing region 20 is formed by an inwardly directed, axially extending crenation 22 which is cut into a wall of the tubular body member 18 to a depth sufficient to expose the pull wire 14. The crenation 22 subtends an angle of greater than 90° but less than 270°. Preferably the crenation 22 subtends an angle slightly less than 180° which, because the deflectable portion 12 is of a plastics material should be sufficient to permit bending in a plane passing through the longitudinal axis of the deflectable portion 12.

The stylet 10 further includes a mounting assembly 23. The mounting assembly 23 comprises an anchoring formation 24 and a mounting member, or carrier, 26 displaceably arranged relative to the anchoring formation 24 to be displaceable in the direction of arrows 28.

A proximal end of the pull wire 14 is mounted fast with the anchoring formation 24. A proximal end of the tube 16 is mounted fast with the carrier 26 so that relative displacement between the carrier 26 and the anchoring formation 24 results in deflection of the deflectable portion 12 in a plane passing through the longitudinal axis of the deflectable portion. More particularly, when the tube 16 is urged distally relative to the anchoring formation 24, deflection of the deflectable portion 12 occurs about the bend-enhancing region 20.

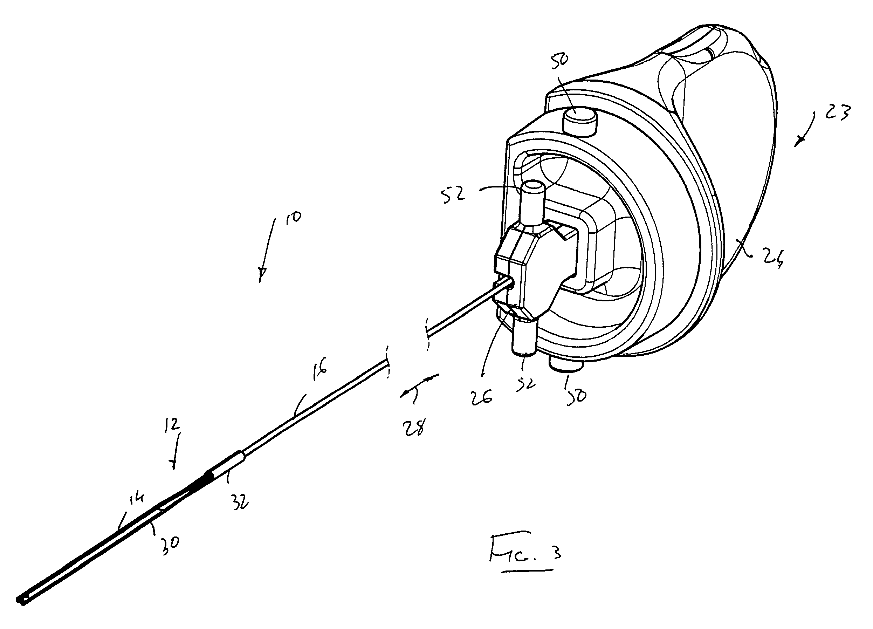

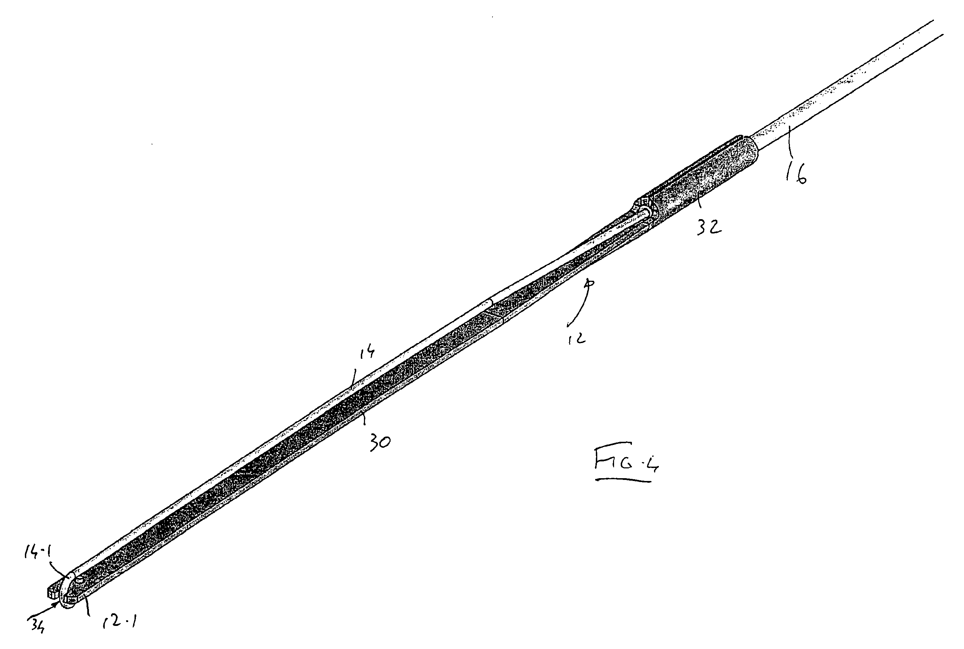

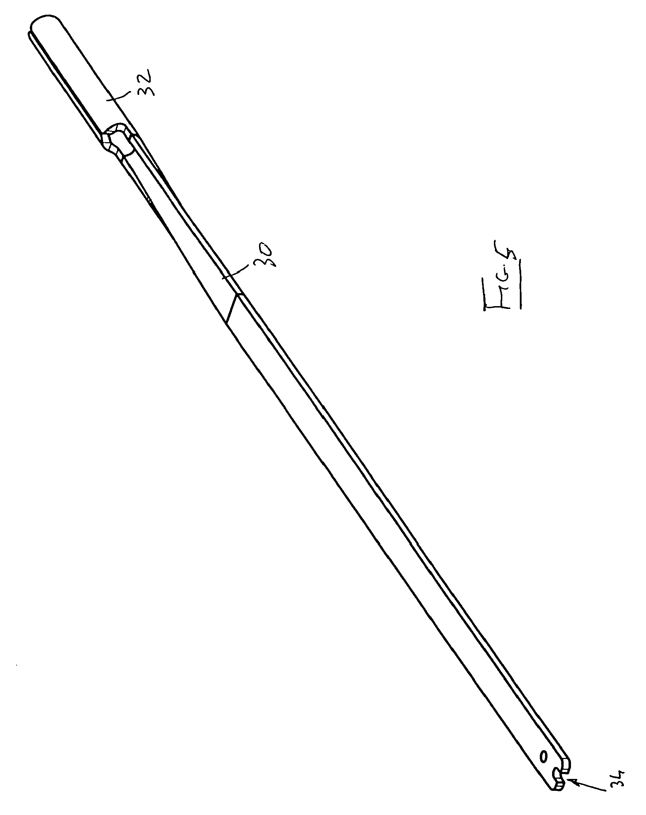

Referring now to Figs. 3-5 of the drawings, a second embodiment of a stylet 10 is illustrated. With reference to the previous drawings, like reference numerals refer to like parts unless otherwise specified. In this embodiment, the deflectable portion 12 comprises an elongate strip-like member 30 shown more clearly in Figs. 4 and 5 of the drawings. The elongate, strip-like member 30 has a proximal boss formation 32 which attaches to the distal end of the tube 16. The pull wire 14 passes over a surface of the strip-like element 30 and the distal end 14.1 of the pull wire 14 is hooked into the distal end 12.1 of the deflectable portion 12 as shown more clearly in Fig. 4 of the drawings. A notch 34 is defined in an end of the strip-like element 30 to retain the distal end 14.1 of the pull wire 14 in position.

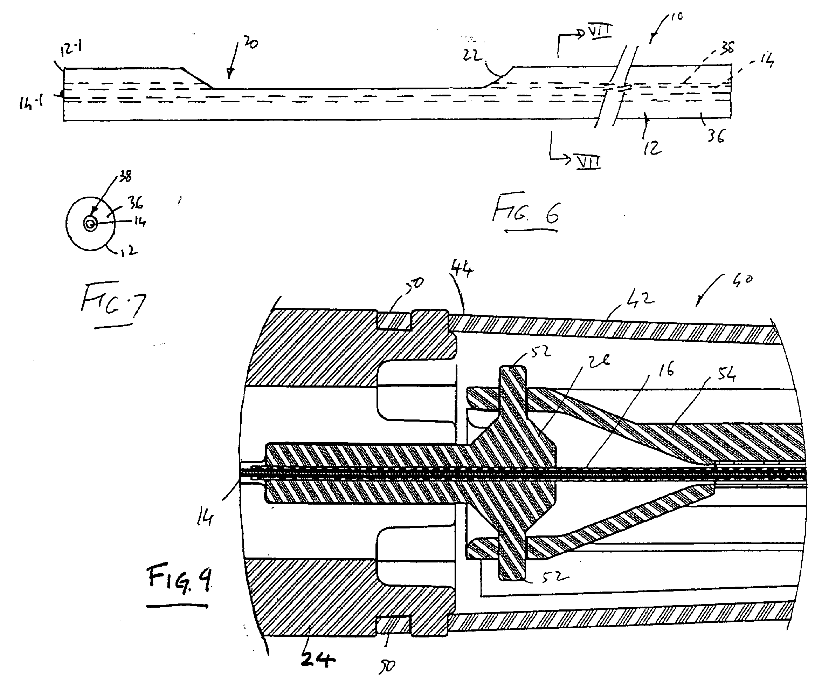

Referring to Figs. 6 and 7 of the drawings, yet a further embodiment of a stylet 10 is illustrated. Once again, with reference to the previous drawings, like reference numerals refer to like parts, unless otherwise specified. In this embodiment, the stylet 10 is a two-part stylet comprising an elongate tubular body member 36 of a PEEK material or a polyimide material and the pull wire 24, the tube 16 being omitted. The elongate tubular body member 36 fulfils the function of the tube 16. Thus, a proximal end of the body member 36 is secured to the carrier 26 of the mounting assembly 23. In this embodiment, the body member 36 extends the full length of the pull wire 14, apart from that part of the pull wire within the anchoring formation 24.

The elongate tubular body member 36 defines a passage 38 through which the pull wire 14 passes. Once again, a distal end 14.1 of the pull wire 14 is fast with a distal end at 12.1 of the deflectable portion 12 of the body member 36. As in the case of the embodiment illustrated in Figs. 1 and 2 of the drawings, the deflectable portion 12 of the body member 36 is formed by the bend-enhancing region 20 being in the form of an inwardly directed, axially extending crenation 22.

To form the crenation 22 in the case of the embodiment shown in Figs. 1 and 2 and in the embodiment shown in Figs. 6 and 7, a tubular member of the plastics material is provided with the passage 38 formed therein. The pull wire 14 is inserted into the passage 38 and is secured to the distal end 12.1 of the deflectable portion 12 to be formed in the body member 18 or 36, as the case may be.

Once the pull wire 14 has been secured in position, a scalpel or other cutting implement is used to form the crenation 22. A wall of the body member 18, 36 is cut away to the depth of the pull wire 14 and the pull wire 14 is used as a guide in forming the crenation 22.

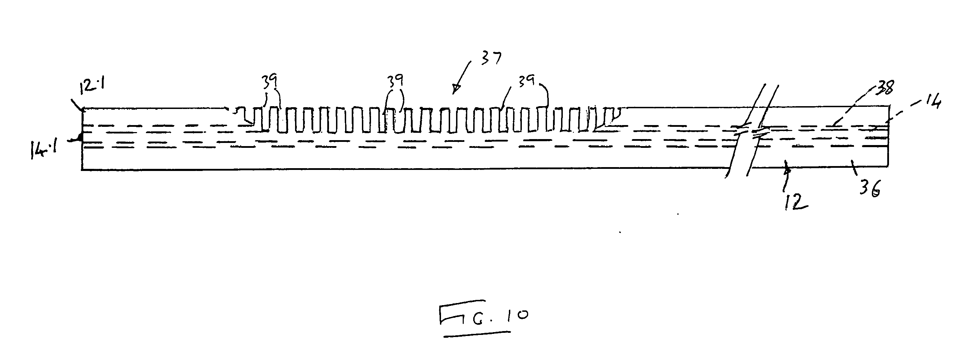

In Fig. 10, yet a further embodiment of a stylet 10 is shown and, once again, with reference to the previous embodiments, like reference numerals refer to like parts unless otherwise specified. In this embodiment, instead of the bend enhancing region 20 of the deflectable portion 12 being defined by the crenation, the bend-enhancing region 20 is defined by a cage 37. The cage 37 comprises a plurality of axially spaced, transverse slots 39 formed in a wall of the body member 36.

In the formation of this embodiment, the pull wire 14 is again inserted into the passage 38 and is used as a guide in the cutting of the slots 39 to the required depth.

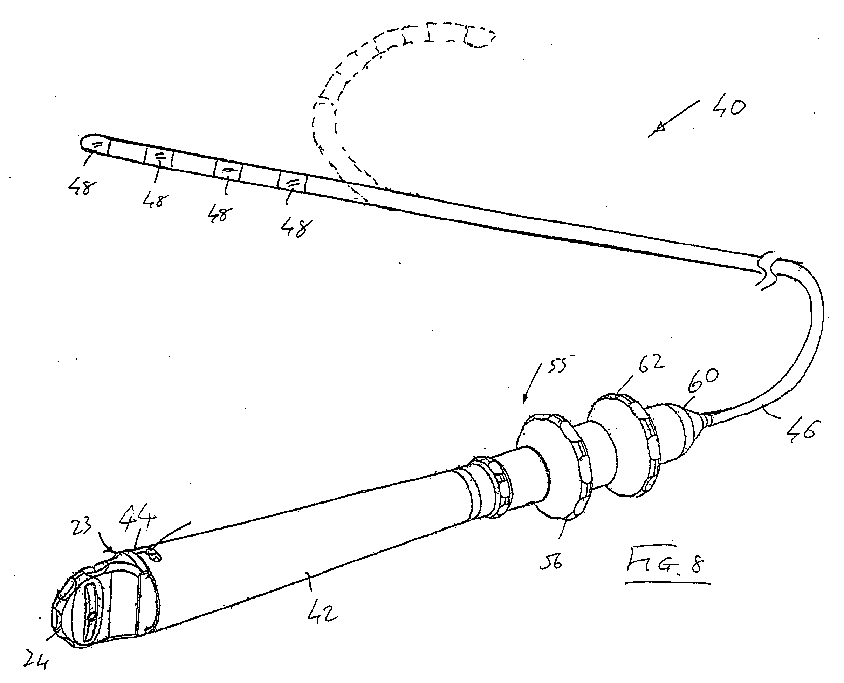

Referring to Fig. 8 of the drawings, an embodiment of a catheter 40 is illustrated. The catheter 40 comprising a handle body 42 having a proximal end 44.

The catheter 40 includes an electrode sheath 46 extending from the handle body 42. A distal end of the electrode sheath carries a plurality of axially spaced electrodes 48. The distal end of the electrode sheath 46 is deflectable into the position shown in dotted lines using the stylet 10, as will be described in greater detail below.

The anchoring formation 24 of the mounting assembly 23 includes an attachment formation in the form of a pair of opposed, radially outwardly extending pins 50 (Fig. 9). The pins 50 engage L-shaped slots at the proximal end 44 of the handle 42, bayonet fashion. Similarly, the carrier 26 includes securing formations in the form of a pair of opposed, radially outwardly extending pins 52 which engage corresponding slots in a slide 54 (Fig. 9) of the handle body 42 of the catheter 40. More particularly, the slide 54 is attached to a part of a deflection control mechanism 55. The deflection control mechanism 55 further includes a deflection control knob 56 axially displaceably arranged on the handle body 42. The slide 54 is fast with the deflection control knob 56. Thus, by urging the deflection control knob 56 in the direction of arrow 58, the tube 16 or the body member 36, as the case may be, of the stylet 10 is urged distally with respect to the pull wire 14 which is fast with the anchoring formation 24 of the mounting assembly 23. This causes the deflectable portion 12 of the stylet 10 to bend about the bend-enhancing region 20 resulting in the deflection as shown in dotted lines in Fig. 8 of the drawings.

The handle body 42 of the catheter 40 further includes a projection control mechanism 60. The projection control mechanism 60 comprises a projection control knob 62 arranged distally of the deflection control knob 56. The electrode sheath 46 is fast with the deflection control mechanism 60. The deflection control mechanism 60 is used to extend the distal end of the electrode sheath 46 relative to a distal of the stylet 10. With such an arrangement, hard to reach places in a patient's heart can be accessed by extending the distal end of the electrode sheath 46 relative to the distal end of the stylet 10.

It is a particular advantage of the invention that a low-cost, single use, deflectable stylet 10 is provided. This renders the catheter 40, in its entirety, disposable as it is made of low-cost materials. Generally, the stylet 10, in order to be deflectable is made from high cost material such as nitinol. However, the Applicant has determined that, surprisingly, by making the stylet out of suitable plastics materials and metals, instead of the high cost material, adequate deflection of the distal end of the electrode sheath 46 can still be obtained and provide the necessary deflectability.

It will be appreciated by persons skilled in the art that numerous variations and/or modifications may be made to the invention as shown in the specific embodiments without departing from the scope of the invention as broadly described. The present embodiments are, therefore, to be considered in all respects as illustrative and not restrictive.

## Claims

1. A single use, deflectable stylet for use in a catheter, the stylet including
a deflectable portion of a flexible material;
an actuator attached to the deflectable portion, manipulation of the actuator relative to the deflectable portion causing deflection of the deflectable portion; and
a stiffening element with the deflectable portion being associated with a distal region of the stiffening element, the stiffening element being sufficiently stiff in torsion to convert proximal rotation into substantially equivalent distal rotation.

2. The stylet of claim 1 in which the deflectable portion is of a synthetic plastics material and in which the stiffening element is of metal.

3. The stylet of claim 1 or claim 2 in which the deflectable portion is an elongate tubular body member defining a passage.

4. The stylet of claim 3 in which the tubular body member defines an axially extending cutaway region in a wall of the body member to facilitate deflection of a distal part of the body member.

5. The stylet of claim 4 in which the cutaway region is closed off by a cage defined by a plurality of longitudinally spaced, transversely extending slots.

6. The stylet of claim 1 or claim 2 in which the stiffening element is a tube and the deflectable portion constitutes a tip portion mounted on a distal end of the tube.

7. The stylet of claim 6 in which the actuator is a pull wire extending through the tube and a tubular body member of the deflectable portion, a distal end of the pull wire being fast with a distal end of the tubular body member.

8. The stylet of claim 7 in which the deflectable portion comprises an elongate, planar element having a mounting formation at its proximal end for mounting to the distal end of the tube.

9. A single use, deflectable stylet for use in a catheter, the stylet including
an elongate, tubular body member of a synthetic plastics material, the plastics material being of a type which permits deflection of at least a part of the body member but which is sufficiently stiff in torsion to convert rotation at a proximal end of the body member into substantially equivalent rotation at a distal end of the body member, the body member defining a lumen;
an axially extending cutaway region defined in a wall of the body member at a distal part of the body member to facilitate deflection of the distal part of the body member, the cutaway region subtending an angle of greater than 90° but less than 270°; and
an actuator received in the lumen of the body member, a distal end of the actuator being fast with a distal end of the body member and relative displacement between the body member and the actuator causing deflection of the body member about the cutaway region of the body member.

10. The stylet of claim 9 in which the body member is of a thermoplastic material, the cutaway region being formed with the actuator in position in the lumen of the body member.

11. The stylet of any one of the preceding claims which includes a mounting assembly for mounting to a handle body of a catheter.

12. A method of fabricating a deflectable stylet, the method including
providing an elongate tubular body member defining a lumen;
inserting an actuator into the lumen of the body member; and
using the actuator as a guide formation, forming an inwardly crenated, axially extending cutaway portion in a wall of the tubular member to form a bend-enhancing region about which the body member is able to deflect.

13. The method of claim 12 which includes forming the cutaway portion with a subtended angle greater than 90° but less than 270°.

14. The method of claim 12 or claim 13 which includes securing a distal end of the actuator fast with a distal end of the body member.

15. The method of any one of claims 12 to 14 which includes providing a stiffening member for the stylet.

16. The method of claim 15 in which the stiffening member is a tube and in which the method includes mounting the tubular body member on a distal end of the tubular member.

17. The method of claim 15 in which the stiffening member is a tube and in which the method includes inserting the tube into the lumen of the body member.

18. The method of claim 15 in which the stiffening member is a tube and in which the method includes inserting the body member into a passage of the tube.
